# EUROPEAN PATENT SPECIFICATION

(11) **EP 1 708 637 B1**
(45) Date of publication and mention of the grant of the patent: **29.09.2010**
(21) Application number: 05702612.2
(22) Date of filing: 07.01.2005
(51) Int. Cl.: A61B 19/00

(54) **DEVICE AND METHOD FOR NAVIGATING A CATHETER**
VORRICHTUNG UND VERFAHREN ZUR NAVIGATION EINES KATHETERS
DISPOSITIF ET PROCEDE DE NAVIGATION D'UN CATHETER

(30) Priority: 20.01.2004 EP 04100160
(43) Date of publication of application: 11.10.2006
(73) Proprietor: Philips Intellectual Property & Standards GmbH, 20099 Hamburg (DE); Koninklijke Philips Electronics N.V., 5621 BA Eindhoven (NL)
(72) Inventor: TIMINGER, Holger Philips, 52066 Aachen (DE); KRÜGER, Sascha Philips, 52066 Aachen (DE); BORGERT, Jörn Philips, 52066 Aachen (DE)
(74) Representative: Volmer, Georg
(86) International application number: PCT/IB2005/050090
(87) International publication number: WO 2005/070318

(56) References cited:
- WO-A-01/20552
- US-A1- 2003 074 011
- US-B1- 6 473 635
- KOENIG A ET AL: "DYNAMIC RECONSTRUCTION FOR RADIOTHERAPY PLANNING" CARS. COMPUTER ASSISTED RADIOLOGY AND SURGERY. PROCEEDINGS OF THE INTERNATIONAL CONGRESS AND EXHIBITION, PROCEEDINGS OF THE INTERNATIONAL SYMPOSIUM ON COMPUTER ASSISTED RADIOLOGY AND SURGERY, 20 June 2002 (2002-06-20), pages 521-526, XP008031712
- SCHAEFFTER T ET AL: "MOTION COMPENSATED PROJECTION RECONSTRUCTION" MAGNETIC RESONANCE IN MEDICINE, ACADEMIC PRESS, DULUTH, MN, US, vol. 41, no. 5, May 1999 (1999-05), pages 954-963, XP001065420 ISSN: 0740-3194

## Description

The invention relates to a device and a method for navigating an instrument, such as, in particular, a catheter or an intervention needle in a body volume (for example, a vessel system or organ) that is subject to a spontaneous movement.

In minimally invasive medical interventions, an instrument, such as, for example, a probe at the tip of a catheter, is pushed through the vessel system of a patient to a point to be investigated or treated. To do this, it is important for the navigation of the instrument and the success of the intervention that the current position of the instrument relative to the vessel system is known as precisely as possible. In this connection, vessel maps are frequently used, that is to say previously obtained two-dimensional or three-dimensional images on which the vessel system is shown in a readily recognizable way. The spatial position and orientation of the instrument determined, for example, with a magnetic locating system can then be marked on the vessel map so that the physician can immediately recognize the location of the instrument that is important for the treatment relative to the vessel system.

A problem in the procedure described is, however, that the vessel system is in many cases (in particular, in the chest or heart region) subject to a constant movement and deformation due to heartbeats and respiration. The current shape and location of the vessel system therefore frequently deviates from its shape and location on the vessel map, with the result that troublesome deviations arise in correlating the current instrument position and instrument orientation with the static vessel map. To compensate for such effects, US 6 473 635 B1 proposes preparing separate vessel maps for various ECG phases and using the respective vessel map corresponding to the current ECG phase during later measurements.

Against this background, the object of the present invention was to provide means for the simplified and, at the same time, as precise navigation as possible of an instrument in a moving body volume of a patient.

This object is achieved by a device having the features of claim 1 and also by a method having the features of claim 10. Advantageous refinements are contained in the subclaims.

The device according to the invention serves to navigate an instrument in a body volume, for example an investigation or treatment device at the tip of a catheter in a vessel system or an intervention needle in an organ. In this connection, the term "vessel system" is to be understood in the present case broadly in the sense of a network of paths in which the instrument may dwell. This term therefore encompasses, in addition to blood vessel systems, for example, also the gastro-intestinal tract system of a patient (in which case the instrument may be in a swallowed probe) or, in the technical field, channels in the interior of a machine. It is to be characteristic of the body volume that it is subject to a spontaneous - preferably cyclic - movement that can be described by a one-dimensional or multidimensional movement parameter. Thus, for example, the (blood) vessel system of a patient is subject to a spontaneous movement that is caused by the heartbeats and that can be characterized with great precision by the respective phase of the electrocardiogram (ECG). The device comprises the following components:
a) A locating device for detecting the current location of the instrument. Here and below, "location" is to be understood in this connection, in particular, as the spatial position and/or the spatial orientation (with three degrees of freedom in each case). The locating device may, for example, be a device that determines the position and/or orientation of the instrument with the aid of magnetic fields or optical methods. The locating device may furthermore be designed to determine the location of a plurality of points of the instrument in order, in this way, to determine, for example, also the orientation or course of a catheter tip.
b) a sensor device for determining the current movement parameters of the spontaneous movement. It may, for example, be an electrocardiograph appliance for measuring the electrocardiogram (ECG) and/or a respiration sensor for determining the respiration phase.
c) A data processing device that is coupled to said locating device and the said sensor device and that comprises a movement model that describes the movement of the body volume as a function of the movement parameter. Typically, the movement model is stored in the form of parameters (data) and/or functions (software) in a memory of the data processing device. Furthermore, the data processing device is designed to calculate a "movement-compensated location" of the instrument with respect to a "current" location, measured with the locating device, of the instrument and to the "current" value, measured in parallel therewith using the sensor device, of the movement parameter. In this connection, "movement-compensated location" denotes that location that is estimated with the movement model and that the instrument would have in a specified reference phase of the spontaneous movement.

The device described makes it possible to track the movement of an instrument in the body volume with respect to a certain, specified reference phase of the spontaneous movement of the body volume. The effect of the spontaneous movement of the body volume on the instrument is compensated for in this connection so that only the relative movement, important for navigation, is left over between instrument and body volume. In order to achieve this objective, the device requires only the movement model stored in the data processing device and also the locating device and the sensor device. A continuous X-ray fluoroscopic observation of the instrument or the preparation of vessel maps from different heartbeat phases is, on the other hand, unnecessary.

In accordance with a preferred refinement of the invention, the data processing device is designed to reconstruct a movement model from measured values for the locations of interpolation nodes from the body volume and from measured values of the respective associated movement parameter. In this approach, the movement model is consequently based on the observed movement of interpolation nodes such as, for example, distinctive vessel bifurcations.

The abovementioned calculation of the movement model is preferably supplemented by an interpolation of the measured movement of the interpolation nodes. That is to say the movement of points situated between the interpolation nodes is calculated with the aid of algorithms, such as, for example, a multiquadric interpolation from the movements of the interpolation nodes. In this connection, the precision of the movement model can be adjusted as desired by means of the density of the network of interpolation nodes.

The measured location values, used for the approach explained above, of interpolation nodes can be determined from a series of three-dimensional images of the body volume. Such images can be obtained, for example, using suitable X-ray or magnetic-resonance devices, wherein the associated movement parameters have each to be determined with respect to the recordings.

In addition or as an alternative thereto, the measured location values of the interpolation nodes may also be locations of the instrument that were determined with the locating device. In that case, the locations, measured for an interpolation node, of the instrument preferably correspond to a state in which no relative movement took place between the instrument and the body volume. For example, the position and, possibly, orientation of a catheter tip can be measured for the duration of a heartbeat phase without forward travel of the catheter, wherein the measurement then describes the movement of an associated interpolation node in the movement model.

In accordance with another development of the invention, the data processing device comprises a memory containing a static image of the body volume. Furthermore, the data processing device is designed to determine the movement-compensated location of the instrument in said static image. In this connection, the reference phase of the spontaneous movement to which the movement-compensated location of the instrument is related is preferably identical to the movement phase that belongs to the static image of the body volume. The static image may be displayed, for example, on a display device, such as a monitor, in which case the associated current location of the instrument can simultaneously be displayed on the image. The static image can consequently serve as a map on which the movement of the instrument may be tracked without the spontaneous movement of the body resulting in this case in disturbances or discrepancies.

The invention furthermore relates to a method of navigating an instrument in a body volume that is subject to a spontaneous movement describable by a movement parameter. The method comprises the following steps:
a) The measurement of the locations of interpolation nodes of the body volume in various phases of the spontaneous movement and also of the associated movement parameters.
b) The reconstruction of a movement model for the body volume from said measured values.
c) The measurement of the ("current") location of the instrument and of the associated ("current") movement parameter.
d) The calculation of the estimated, movement-compensated location of the instrument for a reference phase of the spontaneous movement with the aid of the movement model.

The method described implements in general form the steps that can be executed with a device of the above-described type. With regard to the details, advantages and developments of the method, reference is therefore made to the above description.

These and other aspects of the invention are apparent and will be elucidated with reference to the embodiments described hereinafter.

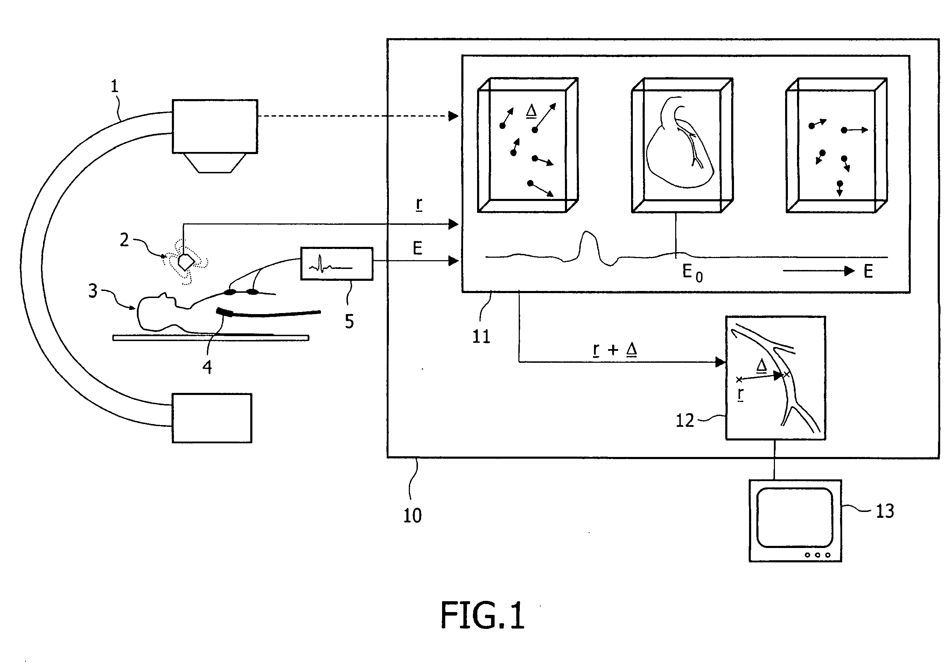

The sole Figure shows diagrammatically the components of a system according to the invention for navigating a catheter in the vessel system of a patient.

The left-hand part of the Figure indicates a situation such as that that occurs, for example, in a catheter investigation of the coronary vessels of a patient 3. In this connection, a diagnostic or therapeutic instrument 4 is pushed forward in the vessel system at the tip of a catheter. The procedure is in many cases continuously observed using an X-ray unit 1 to navigate the catheter in the vessel system. However, this has the disadvantage of a corresponding X-ray exposure for the patient and the investigating staff.

To avoid such exposures, a static vessel map may be used, for example an (X-ray) angiogram obtained while administering a contrast medium, the current position of the instrument 4 being determined using a locating device 2. The locating device 2 may comprise, for example, (at least) a magnetic-field probe at the tip of the catheter with whose aid the strength and direction of a magnetic field is measured that is impressed on the space by a field generator, and this in turn makes possible an assessment of the spatial location (position and orientation) of the catheter. The spatial location of the catheter 4 determined in this way can then be displayed on the static vessel map. A problem in this connection is, however, that there is a severe, essentially cyclic spontaneous movement of the coronary vessels that is caused by the heartbeats and the respiration. Since the vessel map used corresponds to a particular (reference) phase of said movement cycle, whereas the actual instrument location originates, as a rule, from another movement phase, errors arise in the correlation of the instrument location with the static vessel map.

To avoid such errors, the system explained below is proposed. This consists essentially of a data processing device 10 (microcomputer, workstation) with associated devices, such as a central processor, memories, interfaces and the like. The data processing device 10 comprises a movement model I for the vessel system, to be investigated, of the patient 3 in a memory. The movement model 11 describes, with respect to a reference phase E₀ of the heartbeat, the movement field or the vectorial displacement Δ to which the points of the vessel system are subject in the various phases E of the heartbeat. In this connection, the phase of the heartbeat is characterized by a movement parameter E that corresponds to the electrical coronary activity (ECG) that is recorded by an electrocardiograph 5.

With the aid of the movement model 11, it is possible to determine, for a current measured position r and orientation o of the instrument 4 and the associated heartbeat phase E, the displacement vector Δ or the transformation tensor **M**, respectively, that converts the measured position r into an estimated position (r + Δ) of the instrument during the reference phase E₀ or converts the measured orientation into an estimated orientation M·o of the instrument during the reference phase, respectively. This "movement-compensated" position (r + Δ) and orientation can then be displayed on a static vessel map 12 that was obtained during the reference heartbeat phase E₀. The movement-compensated position and orientation of the instrument is situated in this connection on the vessel map 12, as a rule, within the vessel system so that confusing deviations between the instrument location shown and the layout of the vessels do not arise as a result of the heartbeat. The vessel map 12 may be displayed together with the movement-compensated location of the instrument on a monitor 13 in order to enable the physician to navigate the catheter.

To derive the movement model 11, three-dimensional serial recordings of the vessel system are preferably used that have previously been obtained with the aid of the X-ray unit 1, a CT apparatus or with an MRI apparatus. Characteristic points in the vessel system, such as bifurcations, are located in said recordings, which can be done, for example, fully automatically or semi-automatically with suitable segmentation algorithms. It is furthermore assumed that the respective associated phase of the heart cycle E was measured for the individual X-ray recordings. The positions of the interpolation nodes can therefore be correlated with the various heartbeat phases, from which the required displacement vectors A and transformation tensors related to a reference phase E₀ can in turn be calculated. For points in the vessel system that are situated in the vicinity of the interpolation nodes, a suitable interpolation method is preferably used to determine their displacement vectors and/or transformation tensors. This may, for example, involve the use of multiquadric equations (cf. "Multiquadric Equations of Topography and Other Irregular Surfaces", Journal of Geophysical Research, vol. 76:8, pages 1905-191 (1971)) or spline-based methods.

In an alternative approach to obtaining the movement data of interpolation nodes in the vessel system, the movement of the instrument 4 is obtained with the aid of the locating device 2 during phases in which no forward travel of the catheter takes place. In said phases, the observed movement of the instrument 4 is consequently attributable solely to the spontaneous movement of the vessel system. The movement of the instrument 4 can then be correlated with the corresponding heartbeat phases by simultaneously measuring the electrocardiogram and can be used as an interpolation node for the calculation of the movement model 11.

Preferably, the above-described methods for obtaining data for the movement model from three-dimensional (X-ray) recordings and from location data of the instrument 4 are combined with one another to achieve a maximum of precision for the movement model. In this connection, in particular, the movement model 11 can also be supplemented continuously during a current medical intervention by further measurement points obtained with the locating device 2 and the ECG apparatus 5 and extended locally, thereby minimizing errors in the interpolation.

As was already mentioned, the method may also be performed with account being taken of the respiration cycle, a suitable respiration sensor being provided in this case to determine the respiration phase. Compensation for the movement of heartbeat and respiration is likewise possible with the method. In this case, the interpolation nodes are determined not only in the state space of a one-dimensional movement parameter (for example, of the ECG), but also in the two-dimensional state space, for example, consisting of ECG and respiration sensor. Since said state space can only be heavily filled in a finite time or results in an unacceptable prolonging of the measurement time, interpolation nodes are determined by interpolation (for example, multiquadric equations, spline interpolation, etc.) for states not measured.

Furthermore, the above-described method for the navigation of a catheter in a vessel system may also be used in other cases, for example the movement of an intervention needle in the heart.

## Claims

1. A device for navigating an instrument (4) in a body volume that is subject to a spontaneous movement that can be described by a movement parameter (E), comprising
a) a locating device (2) for determining the location (r) of the instrument (4);
b) a sensor device (5) for determining the movement parameter (E);
c) a data processing device (10) coupled to the locating device (2) and the sensor device (5) and comprising a movement model (11) that describes the movement of the body volume as a function of the movement parameter (E), **characterized in that** the data processing device (10) correlates an estimated location (r + Δ) of the instrument in a reference phase (E₀) of the spontaneous movement with measured values of the location (r) of the instrument (4) and of the associated movement parameter (E) with the aid of the movement model (11).

2. A device as claimed in claim 1, **characterized in that** the data processing device (10) is designed to reconstruct the movement model (11) from measured values for the location of the interpolation nodes and for the associated movement parameters (E).

3. A device as claimed in claim 2, **characterized in that** the data processing device (10) is designed to supplement the measured movement of the interpolation nodes in the movement model (11) by interpolation.

4. A device as claimed in claim 2, **characterized in that** the data processing device is designed to determine, in particular from X-ray, CT or MRI recordings, measured values for the location of interpolation nodes from a series of three-dimensional images of the body volume.

5. A device as claimed in claim 2, **characterized in that** the measured values for the location of the interpolation nodes of the body volume correspond to locations (r), measured with the locating device (2), of the instrument (4).

6. A device as claimed in claim 5, **characterized in that** the measured locations (r) of the instrument (4) have been obtained without moving the instrument (4) relative to the body volume.

7. A device as claimed in claim 1, **characterized in that** the data processing device (10) comprises a memory containing a static image (12) of the body volume and is designed to determine the location (r + Δ), estimated for the reference phase (E₀), of the instrument (4) in the static image.

8. A device as claimed in claim 1, **characterized in that** the sensor device comprises an ECG apparatus (5) and/or an apparatus for determining the respiration phase.

9. A device as claimed in claim 1, **characterized in that** the locating device (2) is designed to determine the location of the instrument (4) with the aid of magnetic fields and/or with the aid of optical methods.

## Patentansprüche

1. Vorrichtung zur Navigation eines Instruments (4) in einem Körpervolumen, das einer spontanen Bewegung unterliegt, welche durch einen Bewegungsparameter (E) beschrieben werden kann, wobei die Vorrichtung Folgendes umfasst:
a) eine Lokalisiervorrichtung (2) zum Ermitteln der Position (r) des Instruments (4);
b) eine Sensorvorrichtung (5) zum Ermitteln des Bewegungsparameters (E );
c) eine Datenverarbeitungsvorrichtung (10), die mit der Lokalisiervorrichtung (2) und der Sensorvorrichtung (5) gekoppelt ist und ein Bewegungsmodell (11) umfasst, welches die Bewegung des Körpervolumens als eine Funktion des Bewegungsparameters (E) beschreibt,
**dadurch gekennzeichnet, dass**
die Datenverarbeitungsvorrichtung (10) eine geschätzte Position (r + Δ) des Instruments in einer Referenzphase (E₀) der spontanen Bewegung mit gemessenen Werten der Position (r) des Instruments (4) und des zugehörigen Bewegungsparameters (E) mit Hilfe des Bewegungsmodells (11) korreliert.

2. Vorrichtung nach Anspruch 1, **dadurch gekennzeichnet, dass** die Datenverarbeitungsvorrichtung (10) vorgesehen ist, um das Bewegungsmodell (11) anhand von gemessenen Werten für die Position der Interpolationsknoten und für die zugehörigen Bewegungsparameter (E) zu rekonstruieren.

3. Vorrichtung nach Anspruch 2, **dadurch gekennzeichnet, dass** die Datenverarbeitungsvorrichtung (10) vorgesehen ist, um die gemessene Bewegung der Interpolationsknoten in dem Bewegungsmodell (11) durch Interpolation zu ergänzen.

4. Vorrichtung nach Anspruch 2, **dadurch gekennzeichnet, dass** die Datenverarbeitungsvorrichtung vorgesehen ist, um insbesondere ausgehend von Röntgen-, CT- oder MRT-Aufzeichnungen gemessene Werte für die Position von Interpolationsknoten anhand einer Reihe von dreidimensionalen Bildern des Körpervolumens zu ermitteln.

5. Vorrichtung nach Anspruch 2, **dadurch gekennzeichnet, dass** die gemessenen Werte für die Position der Interpolationsknoten des Körpervolumens Positionen (r) des Instruments (4) entsprechen, welche mit der Lokalisiervorrichtung (2) gemessen wurden.

6. Vorrichtung nach Anspruch 5, **dadurch gekennzeichnet, dass** die gemessenen Positionen (r) des Instruments 4 erlangt wurden, ohne das Instrument (4) relativ zum Körpervolumen zu bewegen.

7. Vorrichtung nach Anspruch 1, **dadurch gekennzeichnet, dass** die Datenverarbeitungsvorrichtung (10) einen Speicher umfasst, der ein statisches Bild (12) des Körpervolumens enthält und vorgesehen ist, um die Position (r + Δ), geschätzt für die Referenzphase (E₀), des Instruments (4) in dem statischen Bild zu ermitteln.

8. Vorrichtung nach Anspruch 1, **dadurch gekennzeichnet, dass** die Sensorvorrichtung ein EKG-Gerät (5) und/oder ein Gerät zum Ermitteln der Atmungsphase umfasst.

9. Vorrichtung nach Anspruch 1, **dadurch gekennzeichnet, dass** die Lokalisiervorrichtung (2) vorgesehen ist, um die Position des Instruments (4) mit Hilfe von Magnetfeldern und/oder mit Hilfe von optischen Methoden zu ermitteln.

## Revendications

1. Dispositif pour la navigation d'un instrument (4) dans un volume corporel sujet à un mouvement spontané pouvant être décrit par un paramètre de mouvement (E), comprenant :
a) un dispositif de localisation (2) pour déterminer la localisation (r) de l'instrument (4) ;
b) un dispositif de capteur (5) pour déterminer le paramètre de mouvement (E) ;
c) un dispositif de traitement de données (10) couplé au dispositif de localisation (2) et au dispositif de capteur (5) et comprenant un modèle de mouvement (11) décrivant le mouvement du volume corporel en fonction du paramètre de mouvement (E), **caractérisé en ce que** le dispositif de traitement de données (10) corrèle une localisation estimée (r + Δ) de l'instrument dans une phase de référence (E₀) du mouvement spontané avec les valeurs mesurées de la localisation (r) de l'instrument (4) et du paramètre de mouvement (E) associé à l'aide du modèle de mouvement (11).

2. Dispositif selon la revendication 1, **caractérisé en ce que** le dispositif de traitement de données (10) est conçu pour reconstruire le modèle de mouvement (11) à partir des valeurs mesurées pour la localisation des noeuds d'interpolation et pour les paramètres de mouvement (E) associés.

3. Dispositif selon la revendication 2, **caractérisé en ce que** le dispositif de traitement de données (10) est conçu pour compléter le mouvement mesuré des noeuds d'interpolation dans le modèle de mouvement (11) par interpolation.

4. Dispositif selon la revendication 2, **caractérisé en ce que** le dispositif de traitement de données (10) est conçu pour déterminer, en particulier à partir de rayons X, CT ou d'enregistrements IRM, les valeurs mesurées pour la localisation des noeuds d'interpolation à partir d'une série d'images tridimensionnelles du volume corporel.

5. Dispositif selon la revendication 2, **caractérisé en ce que** les valeurs mesurées pour la localisation des noeuds d'interpolation du volume corporel correspondent aux localisations (r), mesurées avec le dispositif de localisation (2) de l'instrument (4).

6. Dispositif selon la revendication 5, **caractérisé en ce que** les localisations (r) mesurées de l'instrument (4) ont été obtenues sans déplacer l'instrument (4) par rapport au volume corporel.

7. Dispositif selon la revendication 1, **caractérisé en ce que** le dispositif de traitement de données (10) comprend une mémoire contenant une image statique (12) du volume corporel et est conçu pour déterminer la localisation (r + Δ) estimée pour la phase de référence (E₀) de l'instrument (4) dans l'image statique.

8. Dispositif selon la revendication 1, **caractérisé en ce que** le dispositif de capteur comprend un appareil ECG (5) et/ou un appareil pour déterminer la phase de respiration.

9. Dispositif selon la revendication 1, **caractérisé en ce que** le dispositif de localisation (2) est conçu pour déterminer la localisation de l'instrument (4) à l'aide des champs magnétiques et/ou à l'aide de procédés optiques.
